# EUROPÄISCHE PATENTSCHRIFT

(11) **EP 3 113 749 B1**
(45) Veröffentlichungstag und Bekanntmachung des Hinweises auf die Patenterteilung: **14.10.2020**
(21) Anmeldenummer: 15702803.6
(22) Anmeldetag: 06.02.2015
(51) Int. Cl.: A61K 8/25, A61K 8/73, A61K 8/85, A61Q 19/10

(54) **ABRASIVE HAUTREINIGUNGSMITTEL II**
ABRASIVE SKIN CLEANING PRODUCTS II
PRODUITS DE NETTOYAGE DE LA PEAU ABRASIFS II

(30) Priorität: 05.03.2014 DE 102014204053
(43) Veröffentlichungstag der Anmeldung: 11.01.2017
(73) Patentinhaber: Beiersdorf AG, 20253 Hamburg (DE)
(72) Erfinder: KÖNIG, Sylvia, 21635 Jork (DE); OTTO, Sebastian, 20253 Hamburg (DE); ARGEMBEAUX, Horst, 21465 Wentorf (DE)
(86) Internationale Anmeldenummer: PCT/EP2015/052508
(87) Internationale Veröffentlichungsnummer: WO 2015/132041

(56) Entgegenhaltungen:
- EP-A1- 1 366 737
- WO-A1-2015/028418
- WO-A2-2011/106289
- DE-A1- 4 039 244

## Beschreibung

Die Hautreinigung ist von je her ein Bedürfnis des Menschen. Die Methoden und Mittel zur Hautreinigung haben sich im Laufe der Zeit verändert. Heute besteht die Hautreinigung häufig darin sich mit seifenhaltigen oder seifenartigen Produkten zu waschen, mit Duschzubereitungen zu duschen und mit Badeseifen oder Badezubereitungen zu baden. In der Regel reichen diese Verfahren aus um die Haut zu reinigen und zu erfrischen.

Es gibt jedoch Situationen, in denen eine intensivere Reinigung erforderlich ist. Dies ist zum Beispiel der Fall bei stark verschmutzten Hautoberflächen. Eine solch starke Verschmutzung ist bei manchen Berufsgruppen anzutreffen, z.B. bei Automechanikern. Ölhaltige Verschmutzungen lassen sich oftmals mit gewöhnlichen festen oder flüssigen Seifen oder Handreinigungsmitteln nicht entfernen.

Auch bei einer tiefergehenden Hautreinigung, bei der abgestorbene Hornzellen der oberen Hautschicht abgetragen werden sollen um ein gleichmäßigeres Hautbild zu erhalten, reichen normale Hautreinigungsmittel nicht aus.

In beiden Fällen ist es wünschenswert, dass neben der Reinigung durch Wasser und Tenside eine zusätzliche mechanische Reinigung der Haut erfolgt. Hierzu werden tensidhaltige Reinigungsmittel mit Partikeln versetzt. Diese Partikel sind in der Regel Polyethylenpartikel oder pulverisierte Pflanzenbestandteile wie Obstkerne oder Nussschalen.

Die Polyethylenpartikel werden jedoch nur sehr schlecht bis gar nicht in den Klärwerken biologisch abgebaut. In der Folge akkumulieren sie in den Flüssen und Meeren. Sie stellen damit eine Gefahr für die Fischpopulationen dar.

Auch die Partikel aus pulverisierten Pflanzenbestandteilen weisen Nachteile auf, denn diese Partikel sind anfällig für Verkeimungen. Um diese Verkeimungen zu verhindern werden die Partikel aus pulverisierten Pflanzenbestandteilen häufig mit Gamma-Bestrahlung behandelt, einem Verfahren, dem die Verbraucher jedoch kritisch gegenüber stehen. Darüber hinaus haben die Partikel aus pulverisierten Pflanzenbestandteilen meist eine braune Farbe. Dies hat Auswirkungen auf das kosmetische Endprodukt, das von den Verbrauchern aufgrund von dunkleren Einfärbungen daher als "unkosmetisch" eingestuft wird.

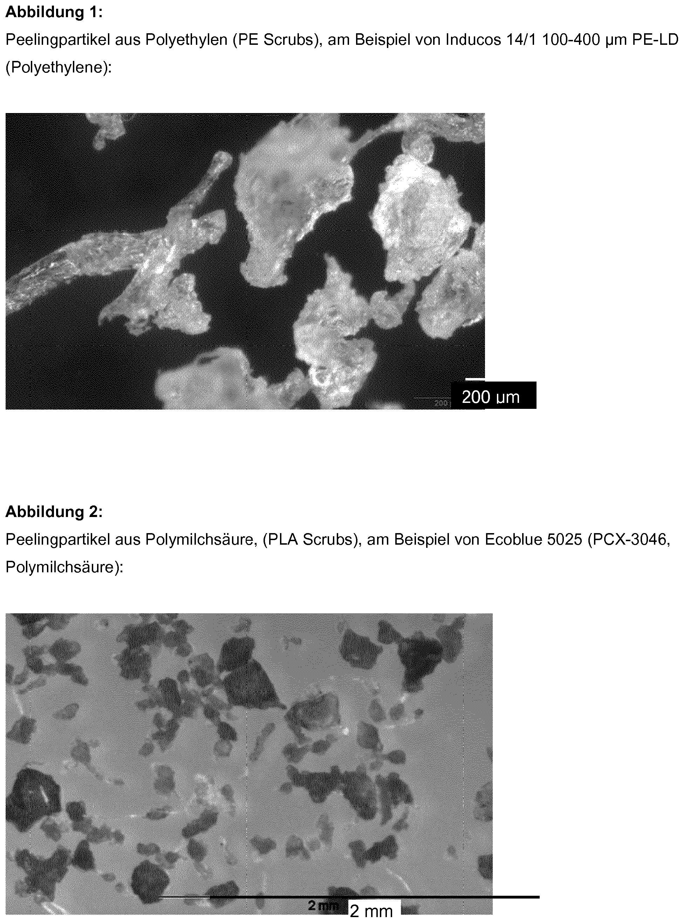

EP 2338962 A1 und EP 2338964 A2 offenbaren abrasive Partikel, die in Reinigungszubereitungen einsetzbar sind. Diese Partikel sind durch unregelmäßige Oberflächen mit teilweise herausragenden Bereichen (Figur 1, EP 2338962 A2) gekennzeichnet. Ein weiterer Nachteil liegt in der Tatsache begründet, dass die Partikel, die als Abrasiva eingesetzt werden, eine recht breite Verteilung in der Partikelgröße aufweisen. Zur Illustration der Partikelgrößenverteilung und der Partikeloberfläche wie sie im Stand der Technik anzutreffen ist, wird in Abbildung 1 eine Aufnahme von Polyethylenpartikeln gezeigt. Es wird deutlich, dass die Oberflächen nicht gleichmäßig rund erscheinen. Darüber hinaus sind Partikel in verschiedenen Größen mit sehr verschiedenen Formen zu erkennen. Das Erscheinungsbild ist inhomogen.

Wenn Partikel mit einer Größe von mehr als 600 µm Durchmesser, insbesondere aber mehr als 850 µm in tensidhaltige Reinigungszubereitungen eingearbeitet werden, ist in vielen Fällen die Sensorik in derartigen Reinigungszubereitungen beeinträchtigt. Beim Auftrag dieser Zubereitungen auf die Haut entsteht ein kratziger Eindruck. Dies ist beim Verbraucher unerwünscht. Darüber hinaus kann die Einarbeitung derartig großer Partikel zu Mikroverletzungen der Haut führen. Diese Mikroverletzungen sind umso problematischer, wenn bei unbeabsichtigtem Kontakt, Augen oder Schleimhäute betroffen sind. Die Mikroverletzungen können besonders ausgeprägt sein, wenn beim Verteilen einer Zubereitung, die derartig große Partikel enthält, die Partikel mehrmals auf der Hautoberfläche hin und her gerieben werden.

WO 2008/107453 A2 offenbart Reinigungskörper aus mindestens 50 % hydriertem Rizinusöl. Diese Partikel weisen eine glatte Oberfläche und eine runde Form auf. Der Einsatz dieser Partikel in Reinigungszubereitungen ist jedoch nicht ohne Probleme möglich, da Hautreinigungsmittel sind in der Regel stark wasserhaltig sind und einer sorgfältigen Konservierung bedürfen, um eine mikrobiologischen Kontamination zu vermeiden. Zur Konservierung sind ausschließlich Konservierungsmittel zugelassen, die in einer Positivliste der Kosmetikverordnung (Deutschland) oder in der Verordnung (EG) Nr. 1223/2009 der EU über kosmetische Mittel genannt werden.

Es gibt wissenschaftliche Studien, die eine Verbindung von bestimmten Konservierungsmitteln und dem Auftreten von Allergien aufzuzeigen versuchen. Ebenso gibt es Untersuchungen, die einen möglichen Einfluss bestimmter Konservierungsmittel auf das Hormonsystem nahelegen. Wie oben ausgeführt sind alle Konservierungsmittel, die in der Kosmetik eingesetzt werden dürfen, in der oben genannten Positivliste aufgeführt. Diese Konservierungsmittel sind als unbedenklich im Einsatz in Kosmetika eingestuft.

Dennoch ist es aus Gründen, die die Galenik oder die Technik bestimmte Rezepturen stabil zu formulieren, betreffen, angezeigt bestimmte Konservierungsmittelklassen bevorzugt zu verwenden.

Es ist also Aufgabe der vorliegenden Erfindung Reinigungszubereitungen zur Verfügung zu stellen, die Peelingkörper enthalten, die eine gute biologische Abbaubarkeit aufweisen und darüber hinaus zu einer Verbesserung in der Sensorik der Reinigungszubereitungen führen.

Weiterhin ist es Aufgabe der vorliegenden Erfindung Reinigungszubereitungen enthaltend Peelingkörper zur Verfügung zu stellen, die ein oder mehrere Konservierungsmittel enthalten, die die Verkeimung der Zubereitungen verhindern, d.h. für antimikrobiell stabile Zubereitungen sorgen.

Weiterhin ist es die Aufgabe, dass Reinigungszubereitungen enthaltend Tenside und Peelingkörper sich durch eine gute Schaumleistung auszeichnen.

Darüber hinaus sollen diese Zubereitungen ein Fließverhalten aufweisen, dass eine gute Entnahme aus dem jeweiligen Packmittel ermöglicht.

Überraschenderweise werden alle vorgenannten Aufgaben gelöst durch tensidhaltige Reinigungszubereitungen, die Tenside und Peelingpartikel enthalten, wobei die Peelingpartikel aus mindestens einer der Substanzen ausgewählt aus der Gruppe, Hydrated Silica, Polymilchsäure und mikrokristalliner Cellulose, bestehen und wobei die Zubereitungen frei sind von Formaldehyd-abspaltenden Konservierungsmitteln, 2-Bromo-2-Nitropropane-1,3-Diol und Butylparaben, gemäss Anspruch 1.

Natürliche Substanzen sind Substanzen natürlichen Ursprungs. Diese Substanzen werden nur Gewinnungsprozessen, Reinigungsprozessen und/oder Konfektionierungsprozessen, d.h. Prozesse zur Generierung der Partikelform, unterworfen. Naturbasierte Substanzen sind Substanzen natürlichen Ursprungs, die vor, während oder nach den Gewinnungsprozessen, Reinigungsprozessen und/oder Konfektionierungsprozessen eine chemische Veränderung erfahren ohne aber ihren natürlichen Charakter zu verlieren.

Kieselsäuren kommen in der Natur als Opal oder in den Zellwänden von Kieselalgen vor. In der Kosmetik gibt es zwei verschiedene Formen, die zum Einsatz kommen, zum einen eine Form, die die INCI-Bezeichnung Silica trägt. Es handelt sich um Kieselsäure mit einem kleinen Teilchendurchmesser. Diese Teilchen haben ein hohes Wasserbindevermögen und fühlen sich auf der Haut stumpf an. Die andere Form trägt die INCI-Bezeichnung Hydrated Silica. Diese Kieselsäure-Teilchen haben einen variablen Wasseranteil und einen größeren Teilchendurchmesser. Sie werden bevorzugt in Zahnpasten eingesetzt und wirken dort als Verdickungsmittel. Die Kieselsäure-Teilchen sind gut biologisch abbaubar. Unter dem Begriff Hydrated Silica werden im Sinne der vorliegenden Erfindung also Kieselsäuren der allgemeinen Formel (SiO₂)ₘ·n H₂O, wobei n und m Werte im Bereich von 1 bis 4 einnehmen können. Fällungskieselsäuren werden ausgehend vom Rohstoff Sand gewonnen. Zunächst wird eine Alkalisilicat-Lösung hergestellt, die mit Mineralsäuren versetzt wird. Die entstehenden Fällungskieselsäuren bilden kolloidale Primärteilchen, die weiter agglomerieren. Schließlich entstehen Aggregate. Vorteilhaft sind amorphe Fällungskieselsäuren, besonders vorteilhaft ist es, wenn die Kieselsäure mikrogranulär vorliegt in Form sphärischer Teilchen. Diese Teilchen entsprechen den Hydrated Silica-Partikeln, die erfindungsgemäß verwendet werden. Weiter ist bevorzugt, wenn die Teilchengröße 320 µm (d50), gemessen mit Hilfe der Laserbeugung in Anlehnung an ISO 787-2, beträgt. Derartige Partikel können beispielsweise bei der Firma Evonik Industries AG erworben werden unter der Bezeichnung Sipernat 2200.

Polymilchsäure ist ein Biopolymer, das aus vielen aneinander gebundenen Milchsäuremolekülen besteht. Diese Biopolymere zeichnen sich dadurch aus, dass sie wasserabweisend sind und damit ist auch verbunden, dass sie nur geringe Mengen an Feuchtigkeit aufnehmen. Die gute biologische Abbaubarkeit in industriellen Kompostieranlagen ist nachgewiesen. Partikel aus Polymilchsäure gibt es in verschiedenen Partikelgrößen, beispielsweise bis 150 µm, bis 297 µm oder bis 850 µm. Polymilchsäurepartikel können nur aus Polymilchsäure oder im Wesentlichen aus Polymilchsäuremolekülen bestehen. Bei den zuletzt genannten Partikeln können Farbstoffe enthalten sein. Derartige Partikel sind beispielsweise von der Firma Micropowders, Inc. zu beziehen. Bevorzugt sind die Partikel Ecoscrub 50PC, Ecosrub 100PC, Ecoblue 5025 und Ecogreen 5025 der Firma Micropowders, Inc. Diese Partikel weisen maximale Partikelgrößen von 297 µm (Ecoscrub 50PC, Ecoblue 5025 und Ecogreen 5025) bzw. 150 µm (Ecosrub 100PC) auf. Besonders bevorzugt sind Partikel mit der Bezeichnung Ecoblue 5025.

Der Begriff Polymilchsäure im Sinne der Erfindung umfasst zwei Ausführungsformen, zum einen bestehend nur aus Polymilchsäure und zum anderen bestehend aus im Wesentlichen Polymilchsäure.

In Sinne der Erfindung bedeutet der Begriff im Wesentlichen, dass die Peelingpartikel zu 90% oder mehr aus der jeweiligen Substanz bestehen.

Mikrokristalline Cellulose besteht aus Cellulose. Um mikrokristalline Cellulose zu gewinnen, wird Cellulose einer sanften Hydrolyse unterworfen, bevorzugt im sauren Bereich. Durch diese Behandlung werden die amorphen Anteile der Cellulose abgebaut. Die verbleibenden Strukturen sind mikrofein, sie werden beispielsweise unter mechanischer Krafteinwirkung in mikrokristalline Cellulose überführt. Cellulose ist biologisch abbaubar. Erfindungsgemäß vorteilhafte Partikel aus mikrokristalliner Cellulose sind beispielsweise bei der Firma J. Rettenmaier & Söhne (JRS) erhältlich. Bevorzugt sind Partikel aus mikrokristalliner Cellulose mit der Bezeichnung Vivapur CS 400, erhältlich bei der Firma J. Rettenmaier & Söhne.

Erfindungsgemäß vorteilhaft sind Zubereitungen, die Peelingpartikel aus im Wesentlichen natürlichen oder naturbasierten Substanzen enthalten, gewählt aus Hydrated Silica oder Polymilchsäure oder mikrokristalliner Cellulose, gemäß Anspruch 1.

Erfindungsgemäß vorteilhaft sind ebenso Zubereitungen, die eine Mischung von Peelingpartikeln enthalten, wobei die Peelingpartikel einer der Substanzen ausgewählt aus der Gruppe, Hydrated Silica, Polymilchsäure und mikrokristalliner Cellulose, bestehen.

Weitere Peelingmittel können in den erfindungsgemäßen Zubereitungen enthalten sein. Bevorzugt sind kugelförmige Peelingpartikel aus mindestens einer wachsartigen Substanz. Wachse im Sinne der vorliegenden Erfindung sind Substanzen, die über 40°C ohne Zersetzung schmelzen. Oberhalb des Schmelzpunktes sind sie nicht fadenziehend und ziemlich niedrig viskos. Nach der Herkunft teilt man Wachse in drei Klassen ein, natürliche Wachse, wie beispielsweise Carnaubawachs und Candelillawachs; chemisch modifizierte Wachse, wie beispielsweise hydrierte Jojobawachse; synthetische Wachse, wie beispielsweise Polyalkylenwachse. Wachsartig bedeutet, dass die eingesetzten Substanzen die oben beschriebenen Eigenschaften aufweisen. Bevorzugt sind die wachsartigen Substanzen auszuwählen aus der Gruppe Carnaubawachs, Candellila Wachs, Sonnenblumenwachs, gehärtetes Rhizinusöl, Reiswachs, gehärtetes Sonnenblumenöl, gehärtetes Sojaöl, Hartparaffin, mikrokristallines Wachs, synthetisches Wachs, gehärtetes Jojobawachs und Stearyl Stearat. Ganz besonders bevorzugt ist sind Peelingpartikel aus gehärtetem Rhizinusöl. Erhältlich sind solche Partikel beispielsweise als WorléeBeads HCO White 60/100 mesh oder WorléeBeads HCO White 20/60 mesh.

Das Gewichtsverhältnis der Peelingpartikel, die aus mindestens einer im Wesentlichen natürlichen oder naturbasierten Substanz, insbesondere aus mindestens einer Substanz ausgewählt aus der Gruppe, Hydrated Silica, Polymilchsäure und mikrokristalliner Cellulose, bestehen zu den Peelingpartikeln bestehend aus mindestens einer wachsartigen Substanz beträgt 1:10 bis 1:10, bevorzugt 3:2 bis 2:3.

Neben den erfindungsgemäß bevorzugt verwendeten Peelingpartikeln können in den erfindungsgemäßen Zubereitungen weitere Peelingmittel enthalten sein. Auch möglich, wenngleich nicht erwünscht, ist der Einsatz von Peelingmitteln, wie beispielsweise Tonerde, Sand, zerstoßene oder zermahlene Kerne von Walnussschalen, Aprikosen-, Pfirsich- und/oder Mandelkernen. Ebenso möglich, wenngleich auch nicht erwünscht ist der Einsatz vollsynthetischer Peelingmittel.

Der Gehalt an Peelingpartikeln in den Reinigungszubereitungen beträgt 0,075 bis 7 Gew.-%, bezogen auf das Gesamtgewicht der Zubereitung.

Suspendierte Partikel, die eine Peelingfunktion in der Zubereitung erfüllen, werden als Peelingpartikel oder Peelingkörper bezeichnet. Die Begriffe Peelingpartikel, Peelingmittel oder Peelingkörper werden in dieser Offenbarung synonym verwendet.

Die erfindungsgemäßen Hautreinigungsmittel, die Peelingpartikel und Tenside enthalten, sind stark wasserhaltig und damit keimanfällig. Derartige Hautreinigungsmittel bedürfen einer ausgewogenen Konservierung. Konservierungsmittel können, wie schon beschrieben, in verschiedene Klassen eingeteilt werden.

Zur Gruppe der halogenorganischen Konservierungsmittel wird beispielsweise 2-Bromo-2-Nitropropane-1,3-Diol gerechnet, ebenso 5-Chlor-2-(2,4-dichlorphenoxy)-phenol (Triclosan), lodopropynyl Butylcarbamate, Methylchloroisothiazolinone, Methyldibromo Glutaronitrile (MDGN), Chloracetamide, Dichlorphenyl-Imidazoldioxolan und andere. Die erfindungsgemäßen Zubereitungen sind frei von 2-Bromo-2-Nitropropane-1,3-Diol. Bevorzugt ist es, dass die erfindungsgemäßen Zubereitungen frei sind von halogenorganischen Verbindungen.

Formaldehydabspalter sind Substanzen, die Formaldehyd abspalten und als Konservierungsmittel zum Einsatz kommen können. Es gibt eine ganze Reihe von derartigen Substanzen, in der Kosmetik werden häufiger Substanzen der Gruppe Diazolidinyl Urea (Germall II®), Imidazolidinyl Urea (Germall 115®), DMDM Hydantoin, MDM Hydantoin und seltener Quaternium 15 verwendet. Die erfindungsgemäßen Zubereitungen sind frei von Formaldehyd-abspaltenden Substanzen.

Butylparaben wird zur Gruppe der Paraben-haltigen Konservierungsmittel gerechnet. Die allgemeine Strukturformel der Parabene ist im Folgenden dargestellt:

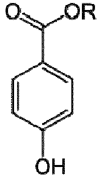

R bedeutet Alkylseitenkette, die aus einer unverzweigten oder verzweigten Kette von 1 bis 5 Kohlenstoffatomen oder aus aromatischen Resten bestehen kann. Bekannte Parabene sind Methyl-, Ethyl-, Propyl- Butylparaben, ebenso wie Isopropyl-, Isobutyl-, Pentyl- und Phenylparaben. Benzylparaben ist in kosmetischen Mitteln als Konservierungsmittel nicht zugelassen. Die erfindungsgemäßen Zubereitungen sind frei von Butylparaben. Es ist bevorzugt, dass die erfindungsgemäßen Zubereitungen frei sind von Parabenen, die eine Alkylseitenkette von mehr als 3 Kohlenstoffatomen aufweisen. In bestimmten Ausführungsformen ist weiter bevorzugt, dass die erfindungsgemäßen Zubereitungen frei sind von Parabenen der Gruppe Methyl-, Ethyl-, Propyl-, Isopropyl-, Isobutyl-, Pentyl- und Phenylparaben.

In Sinne der Erfindung bedeutet frei von, dass nur 0,001 Gew.-% von den entsprechenden Substanzen enthalten sind. Es werden keine der entsprechenden Substanzen den Zubereitungen zugegeben. Die Gehalte sind lediglich bedingt durch die Zugabe von weiterer Bestandteile in die Zubereitungen, die diese Substanzen, insbesondere Konservierungsstoffe, enthalten können.

Es ist erfindungsgemäß, wenn die Zubereitungen als Konservierungsmittel Benzoesäure und/oder ein Salz der Benzoesäure enthalten.

Die Konservierungsmittel aus der Gruppe der Parabene werden mit einem Gehalt von 0,02 bis 0,8 Gew.-%, bevorzugt 0,2 bis 0,4 Gew.-%, bezogen auf das Gesamtgewicht der Zubereitung eingesetzt. Die Konservierungsmittel aus der Gruppe der konservierend wirkenden Säuren, wie beispielsweise Benzoesäure und/oder Salicylsäure, und/oder ihre Salze werden mit einem Gehalt von 0,04 bis 1,0 Gew.-%, bevorzugt 0,45 bis 0,6 Gew.-%, bezogen auf das Gesamtgewicht der Zubereitung eingesetzt.

Die erfindungsgemäßen Hautreinigungszubereitungen sind dadurch gekennzeichnet, dass sie Tenside enthalten.

Es ist bevorzugt, dass die Tenside ausgewählt werden aus der Gruppe der anionischen, amphoteren und nichtionischen Tenside. In den erfindungsgemäßen Zubereitungen ist mindestens ein Tensid aus der angeführten Gruppe enthalten.

Es ist weiter bevorzugt, dass die anionischen Tenside Alkali- und/oder Ammoniumsalze der Alkylsulfate und/oder Alkylethersulfate sind. Besonders bevorzugt sind Alkylsulfate und/oder Alkylethersulfate mit Alkylresten mit einer Kettenlänge von 8 bis 16 Kohlenstoffatomen. Insbesondere bevorzugt sind Alkylsulfate und/oder Alkylethersulfate mit Alkylresten mit einer Kettenlänge von 10 bis 14 Kohlenstoffatomen. Ganz insbesondere bevorzugt sind Alkylsulfate und/oder Alkylethersulfate mit Alkylresten mit einer Kettenlänge von 11 bis 13 Kohlenstoffatomen.

Es ist weiter bevorzugt, dass die amphoteren Tenside Alkyl- und/oder Alkylamidopropylbetaine sind. Besonders bevorzugt sind Alkyl- und/oder Alkylamidopropylbetaine mit Alkylresten mit einer Kettenlänge von 8 bis 16 Kohlenstoffatomen. Insbesondere bevorzugt sind Alkyl- und/oder Alkylamidopropylbetaine mit Alkylresten mit einer Kettenlänge von 10 bis 14 Kohlenstoffatomen. Ganz insbesondere bevorzugt sind Alkyl- und/oder Alkylamidopropylbetaine mit Alkylresten mit einer Kettenlänge von 11 bis 13 Kohlenstoffatomen.

Es ist auch besonders bevorzugt, dass die Alkyl- und/oder Alkylamidopropylbetaine Alkylgruppen aufweisen, die aus der Aufarbeitung von Kokosöl zu Tensiden stammen. Insbesondere bevorzugt ist, dass die Alkyl- und/oder Alkylamidopropylbetaine Cocco-Betaine und/oder Cocoamidopropylbetaine sind.

Es ist weiter bevorzugt, dass nichtionischen Tenside ethoxilierte Glycerinester sind. Besonders bevorzugt sind ethoxilierte Glycerinester mit Alkylresten mit einer Kettenlänge von 8 bis 16 Kohlenstoffatomen. Insbesondere bevorzugt sind ethoxilierte Glycerinester mit Alkylresten mit einer Kettenlänge von 10 bis 14 Kohlenstoffatomen. Ganz insbesondere bevorzugt sind ethoxilierte Glycerinester mit Alkylresten mit einer Kettenlänge von 11 bis 13 Kohlenstoffatomen. Noch weiter bevorzugt ist es, dass die ethoxilierten Glycerinester PEG-7 Glyceryl Cocoate sind.

Erfindungsgemäß vorteilhaft beträgt der Aktivgehalt der Tenside in den Hautreinigungszubereitungen 5 - 18 Gew.-%, bevorzugt 10 - 15 Gew.-%, insbesondere bevorzugt 10 bis 13 Gew.-%, bezogen auf das Gesamtgewicht der Zubereitung.

Es können weitere Tenside in den erfindungsgemäßen Zubereitungen zum Einsatz kommen, dies können anionische und/oder amphotere und/oder nichtionische Tenside sein.

Vorteilhaft zu verwendende anionische Tenside sind
Acylaminosäuren (und deren Salze), wie
1. Acylglutamate, beispielsweise Natriumacylglutamat, Natrium Cocoylglutamat, Di-TEApalmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat.
Carbonsäuren und Derivate, wie
1. Carbonsäuren, beispielsweise Laurinsäure, Aluminiumstearat, Magnesiumalkanolat und Zinkundecylenat, Stearinsäure/-salz, Palmitinsäure/-salz,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-Phosphat,
Sulfonsäuren und Salze, wie
1. Acyl-isethionate, z.B. Natrium-/ Ammoniumcocoyl-isethionat, Natrium Lauryl Methylisethionate,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium-Ammonium- und TEA-Cocosulfat.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Um den erfindungsgemäßen Zubereitungen rückfettende Eigenschaften zu verleihen können beispielsweise Öle in die erfindungsgemäßen Zubereitungen eingearbeitet werden.

Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat,lsopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C12-15-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Halistar AB*) und/oder Diethylhexylnaphthalat (*Hallbrite TQ oder Corapan TQ von H&R).*

Auch beliebige Abmischungen solcher Öl- und Wachskomponenten sind vorteilhaft im Sinne der vorliegenden Erfindung einzusetzen.

Ferner können optional auch unpolare Öle enthalten sein, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen. Diese unpolaren Öle liegen nicht in Partikelform vor.

Ferner können die Zubereitungen optional einen Gehalt an cyclischen oder linearen Silikonölen aufweisen.

Besonders vorteilhaft ist beispielsweise die Verwendung von Coco-Caprylate/Caprate.

Erfindungsgemäße Zubereitungen können ein oder mehrere polymere Strukturanten enthalten. Die polymeren Strukturanten können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi ausgewählt werden; besonders vorteilhaft ist Xanthangummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Vorteilhaft sind außerdem Taurate, z.B. Ammonium Acryloyldimethyltaurat / VP Copolymer.

Unter den Polyacrylaten befinden sich z.B. Carbopol Typen der Firma Lubrizol (Carbopol 980, 981, 1382, 5984, 2984, ETD 2001, ETD 2020, ETD 2050, Ultrez-10 oder Pemulen TR1 & TR2), Acrylates Copolymer (Handelsname Carbopol Aqua SF-1) oder Acrylates Crosspolymer-4 (Handelsname: Carbopol Aqua SF-2).

Unter den Polymeren befinden sich z.B. Polyacrylamide (Seppigel 305), Polyvinylalkohole, PVP, PVP / VA Copolymere, Polyglycole.

Erfindungsgemäß besonders bevorzugt ist ein Acrylic Acid/VP Crosspolymer von der Firma ASI für Reinigungszubereitungen enthaltend Kristall-stabilisierende Polyole.

Erfindungsgemäß sind auch Substanzen, die üblicherweise eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure und/oder aus der Gruppe Aminomethylpropanol, Natronlauge, Kalilauge und Triethanolamin.

Optional können, wenn erforderlich, die in der Kosmetik üblichen Zusatz- und Hilfsstoffe in die Zubereitungen eingearbeitet werden, wie z.B. desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfüms, weichmachende Substanzen, anfeuchtende und/oder feuchthaltende Substanzen, Antioxidantien, UV-Filtersubstanzen, Sensorikadditive, Filmbildner, Wirkstoffe oder andere übliche Bestandteile einer kosmetischen Formulierung wie Alkohole, Polyole, Schaumstabilisatoren, Elektrolyte, organische Lösungsmittel oder Silikonderivate.

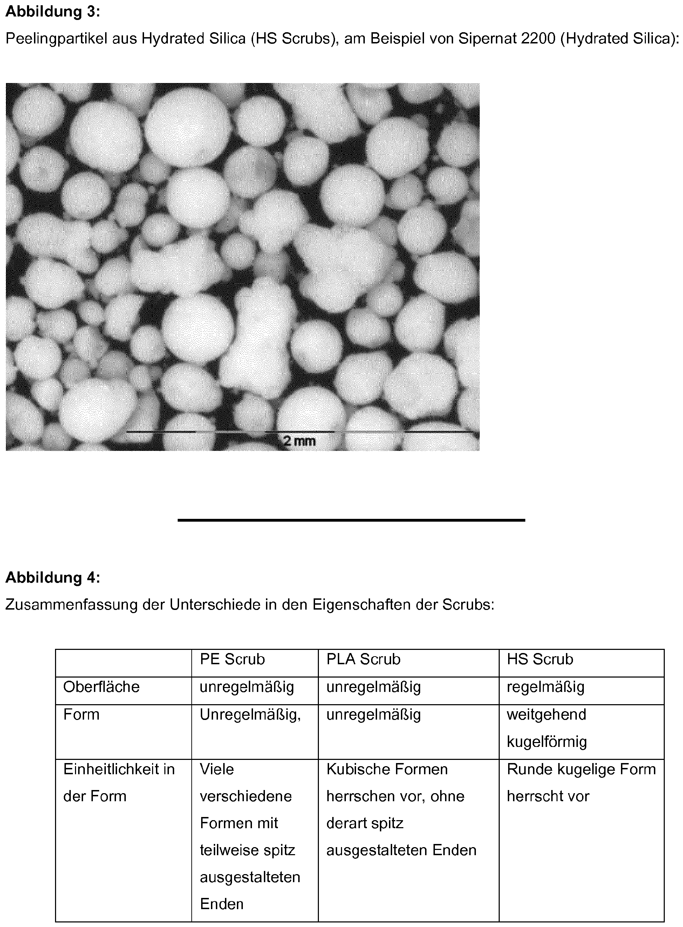

Die Abbildungen 1 bis 3 zeigen mikroskopische Aufnahmen von Peelingpartikeln wie sie aus dem Stand der Technik bekannt sind und Peelingpartikeln, die in den erfindungsgemäßen Zubereitungen zum Einsatz kommen. Die Oberfläche der Peelingpartikel aus Polyethylen (PE Scrubs), am Beispiel von Inducos 14/1 100-400 µm PE-LD (Polyethylene), die in Abbildung 1 gezeigt werden, weist ein unregelmäßiges Aussehen auf. Ebenso ist eine große Formenvielfalt, kompakt bis stabförmig, zu erkennen. Die Peelingpartikel aus Polymilchsäure, (PLA Scrubs), am Beispiel von Ecoblue 5025 (PCX-3046), die in Abbildung 2 dargestellt werden, zeigen eine geringere Formenvielfalt, stabförmige Elemente sind weniger stark vertreten. Peelingpartikel aus Hydrated Silica (HS Scrubs), am Beispiel von Sipernat 2200 (Hydrated Silica), siehe Abbildung 3, zeigen eine glatte Oberfläche, nahezu rund Formen und eine Größenberteilung in einem engen Bereich.

Je gleichmäßiger in Bezug auf Form, Oberfläche und Größenverteilung eine Partikelpopulation ist, desto angenehmer wird die Sensorik bei der Anwendung von Hautreinigungsmitteln empfunden.

Abbilung 4 zeigt eine Zusammenfassung der Daten zu den einzelnen Partikelpopulationen. Zusammenfassend ist festzustellen, dass Partikel aus Hydrates Silica aufgrund der Gleichmäßigkeit in Bezug auf Form, Oberfläche und Größenverteilung einen Beitrag zu der angenehmen Sensorik der erfindungsgemäßen Hautreinigungsmittel leistet. Auch Partikel aus Polymilchsäure, wahlweise in Kombination mit anderen erfindungsgemäß bevorzugten Partikeln tragen zu einer verbesserten Sensorikleistung der erfindungsgemäßen Hautreinigungsmittel bei.

Die Mengenangaben in der vorliegenden Erfindung beziehen sich auf Aktivgehalte und sind immer Angaben in Gew.-%; es sei denn, es werden ausdrücklich abweichende Angaben gemacht. Die Mengenangaben in Gew.-% in den nachfolgenden Beispielen sind Einsatzkonzentrationen der verwendeten Komponenten oder im Handel erhältlichen Vormischungen.

### Beispiele:

| **Duschgele:** | | **Gew.-%** | | | | | |
|---|---|---|---|---|---|---|---|
| **INCI** | **Rohstoff-**/ **Handelsname** | **1** | **2** | **3** | **4** | **5** | **6** |
| PEG-40 Hydrogenated Castor Oil | Sympatens TRH/400, Kolb | 0.40 | 0.40 | 0.40 | 0.40 | 0.40 | 0.40 |
| Parfum | Parfum | 0.80 | 0.80 | 0.80 | 0.80 | 0.80 | 0.80 |
| Aqua + Sodium Hydroxide | Natronlauge 45% | 0.20 | 0.30 | 0.20 | 0.30 | 0.20 | 0.20 |
| Citric Acid | CITRIC ACID MONOHYDRATE FINE GRANULAR | 0.60 | 0.60 | 0.60 | 0.60 | 0.60 | 0.60 |
| Aqua + Styrene/Acrylates Copolymer | Opulyn 301, Dow Chemical | 1.00 | 1.20 | 0.80 | 1.10 | 1.00 | 1.20 |
| Polylactic Acid, CI42090 | PCX-3046, Micro Powders | 0.20 | 0.05 | | | | 0.30 |
| Microcrystalline Cellulose | Vivapur CS 400, JRS | 1.00 | | 0.80 | 1.20 | 2.00 | |
| Hydrated Silica | Sipernat 2200, Evonik | | 1.00 | | | | 1.00 |
| Oryza Sativa Bran Wax | Natureblue 20RS, Micro Powders | | | | 3.00 | | |
| Cera Microcristallina | WorléeBeads CMSnow White 40/60 mesh, Worlee | | | 1.00 | | 1.50 | |
| Synthetic Wax | WorléeBeads SYN Snow White 40/60 mesh, Worlee | | | | 1.00 | | |
| Hydrogenated Castor Oil | Worlee Beads HCO 20/60mesh, Worlee | 3.50 | 2.00 | 2.00 | 1.00 | | 0.50 |
| Lactose + Cellulose + CI 77007 + Hydroxypropyl Methylcellulose | Unispheres UE-507, Induchem | 0.10 | 0.10 | 0.10 | 0.10 | 0.10 | 0.10 |
| Sodium Benzoate | Natrium Benzoat | 0.45 | 0.45 | 0.45 | 0.45 | 0.45 | 0.45 |
| Aqua + Acrylates Copolymer | Carbopol Aqua SF-1 Polymer, Lubrizol Advanced Materials | 4.50 | 5.00 | 4.50 | 5.50 | 4.50 | 5.00 |
| PEG-200 Hydrogenated Glyceryl Palmate + Aqua | Rewoderm LI 520-70, Evonik Industries | 0.20 | 0.10 | 0.20 | 0.10 | 0.20 | 0.20 |
| Aqua | Wasser | 65.05 | 71.00 | 66.15 | 62.45 | 66.25 | 67.25 |
| Sodium Laureth Sulfate + Aqua | Texapon N 70, ca. 70%, BASF Personal Care and Nutrition | 13.00 | 10.00 | 11.00 | 9.00 | 12.00 | 13.00 |
| Aqua + Cocamidopropyl Betaine + Glycerin | Tego-Betain F 50, Evonik Industries | 9.00 | 7.00 | 11.00 | 13.00 | 10.00 | 9.00 |

| **Gesichtsreinigungsgele** | | **Gew.- %** | | | | | |
|---|---|---|---|---|---|---|---|
| **INCI** | **Rohstoff-**/ **Handelsname** | **1** | **2** | **3** | **4** | **5** | **6** |
| Parfum | Parfum | 0.10 | 0.10 | 0.10 | 0.10 | 0.10 | 0.10 |
| Glycerin + Aqua | Refined Glycerin, 99% | 1.50 | 1.50 | 1.70 | 2.00 | 1.50 | 1.80 |
| Aqua + Sodium Hydroxide | Natronlauge 45%, | 0.77 | 0.77 | 0.77 | 0.77 | 0.77 | 0.77 |
| Nylon-11 | Rilsan T Nat BHV Cos, Arkemap | 2.50 | 2.90 | 2.90 | 2.70 | 2.90 | 2.80 |
| Copernica Cerifera Cera | NATUREBEAD B20, Impag | 0.50 | | | | | |
| Candilla Cera | Candelillawachsperlen | | 0.20 | | | | 1.00 |
| Helianthus Annuus Seed Cera | Sonnenblumenwachsperlen | | | 0.20 | | | |
| Oryza Sativa Bran Wax | Natureblue 20RS, Micro Powders | | | | 0.40 | | |
| Polylactic Acid, CI42090 | PCX-3046, Micro Powders | 0.20 | 0.05 | | | | 0.30 |
| Microcrystalline Cellulose | Vivapur CS 400, JRS | 0.80 | | 0.80 | 1.10 | | |
| Hydrated Silica | Sipernat 2200, Evonik | | 0.55 | | | | 1.00 |
| Oryza Sativa Bran Wax | Natureblue 20RS, Micro Powders | | | | 3.00 | | |
| Cera Microcristallina | WorléeBeads CMSnow White 40/60 mesh, Worlee | | | 1.00 | | 1.50 | |
| Synthetic Wax | WorléeBeads SYN Snow White 40/60 mesh, Worlee | | 0.40 | | | | |
| Hydrogenated Castor Oil | Worlee Beads HCO 20/60mesh, Worlee | 0.50 | | | 1.00 | | 0.50 |
| Lactose + Cellulose + CI 77007 + Hydroxypropyl Methylcellulose | Unispheres UE-507, Induchem | 0.60 | 0.40 | 0.40 | 0.40 | 0.40 | 0.40 |
| Methyl para ben | Solbrol M, Lanxess | 0.40 | 0.30 | 0.30 | 0.35 | 0.30 | 0.35 |
| Phenoxyethanol | Phenoxyethanol P5, Omya Peralta | 0.80 | 0.70 | 0.70 | 0.80 | 0.70 | 0.80 |
| Acrylates/C10-30 Alkyl Acrylate Crosspolymer | Carbopol 1382, Lubrizol Advanced Materials | 1.00 | 0.80 | 0.90 | 0.85 | 0.90 | 0.92 |
| Aqua | Wasser | 88.53 | 89.73 | 88.73 | 84.93 | 89.23 | 87.46 |
| Sodium Laureth Sulfate + Aqua | Texapon N 70, ca. 70%, BASF Personal Care and Nutrition | 1.20 | 1.00 | 0.90 | 1.00 | 1.10 | 1.20 |
| Lauryl Glucoside + Aqua | Plantacare 1200 UP, BASF Personal Care and Nutrition | 0.60 | 0.60 | 0.60 | 0.60 | 0.60 | 0.60 |

| **Flüssigseife** | | **Gew.-%** | | | | | |
|---|---|---|---|---|---|---|---|
| **INCI** | **Rohstoff-**/ **Handelsname** | **1** | **2** | **3** | **4** | **5** | **6** |
| PEG-40 Hydrogenated Castor Oil | Sympatens TRH/400, Kolb | 0.60 | 0.70 | 0.50 | 0.60 | 0.70 | 0.70 |
| PEG-7 Glyceryl Cocoate | Cetiol HE, BASF Personal Care and Nutrition | 1.50 | 1.50 | 1.50 | 1.50 | 1.50 | 1.50 |
| Parfum | Parfum | 1.00 | 1.00 | 1.00 | 1.00 | 1.00 | 1.00 |
| Aqua + Sodium Hydroxide | Natronlauge 45% | 0.44 | 0.44 | 0.44 | 0.44 | 0.44 | 0.44 |
| Polylactic Acid, CI42090 | PCX-3046 | 0.20 | 0.05 | | | | 0.30 |
| Microcrystalline Cellulose | Vivapur CS 400, JRS | 1.00 | | 0.80 | 1.20 | 2.00 | |
| Hydrated Silica | Sipernat 2200, Evonik | | 1.00 | | | | 1.00 |
| Oryza Sativa Bran Wax | Natureblue 20RS, Micro Powders | | | | 3.00 | | |
| Cera Microcristallina | WorléeBeads CMSnow White 40/60 mesh, Worlee | | | 1.00 | | 1.50 | |
| Synthetic Wax | WorléeBeads SYN Snow White 40/60 mesh, Worlee | | | | 1.00 | | |
| Hydrogenated Castor Oil | WorléeBeads Jojoba Snow White40/60 mesh, Worlee | 3.50 | 2.00 | 2.00 | 1.00 | | 0.50 |
| Lactose + Cellulose + CI 77007 + Hydroxypropyl Methylcellulose | Unispheres UE-507, Induchem | 0.10 | 0.10 | 0.10 | 0.10 | 0.10 | 0.10 |
| Methyl para ben | Solbrol M, Lanxess | 0.40 | 0.35 | 0.45 | 0.30 | 0.35 | 0.40 |
| Phenoxyethanol | Phenoxyethanol P5, Omya Peralta | 0.90 | 1.00 | 0.90 | 1.00 | 1.00 | 0.90 |
| Ethylparaben | Ethylparaben, Schütz & Co. | 0.45 | 0.45 | 0.35 | 0.45 | 0.45 | 0.45 |
| Aqua + Acrylates Copolymer | Carbopol Aqua SF-1 Polymer, Lubrizol Advanced Materials | 6.50 | 7.00 | 6.00 | 6.50 | 6.70 | 6.90 |
| Aqua | Wasser | 59.41 | 60.41 | 60.96 | 57.91 | 60.26 | 61.81 |
| Sodium Laureth Sulfate + Aqua | Texapon N 70, ca. 70%, BASF Personal Care and Nutrition | 9.00 | 7.00 | 11.50 | 9.50 | 13.00 | 9.50 |
| Aqua + Cocamidopropyl Betaine + Glycerin | Tego-Betain F 50, Evonik Industries | 13.00 | 15.00 | 10.50 | 12.50 | 9.00 | 12.50 |
| Aqua + Disodium Cocoyl Glutamate + Propylene Glycol | Protelan AGL 95/C, Zschimmer & Schwarz | 2.00 | 2.00 | 2.00 | 2.00 | 2.00 | 2.00 |

## Patentansprüche

1. Hautreinigungszubereitungen enthaltend Tenside und Peelingpartikel, wobei die Peelingpartikel aus mindestens einer Substanz ausgewählt aus der Gruppe, Hydrated Silica, Polymilchsäure und mikrokristalliner Cellulose, bestehen,
wobei die Zubereitungen frei sind von Formaldehyd-abspaltenden Konservierungsmitteln, 2-Bromo-2-Nitropropane-1,3-Diol und Butylparaben,
wobei als Konservierungsmittel Benzoesäure und/oder ein Salz der Benzoesäure enthalten sind und
wobei der Gehalt an Peelingpartikeln in den Reinigungszubereitungen 0,075 bis 7 Gew.-%, bezogen auf das Gesamtgewicht der Zubereitung beträgt.

2. Reinigungszubereitungen nach Anspruch 1, **dadurch gekennzeichnet dass**, die Peelingpartikel nur aus jeweils einer im Wesentlichen natürlichen oder naturbasierten Substanz bestehen, insbesondere jeweils nur Hydrated Silica oder Polymilchsäure oder mikrokristalliner Cellulose.

3. Reinigungszubereitungen nach Anspruch 1, **dadurch gekennzeichnet, dass** die Zubereitungen eine Mischung von Peelingpartikeln enthalten, wobei die Peelingpartikel aus im Wesentlichen natürlichen oder naturbasierten Substanzen, insbesondere aus Substanzen ausgewählt aus der Gruppe Hydrated Silica, Polymilchsäure und mikrokristalliner Cellulose, bestehen.

4. Zubereitung nach wenigstens einem der vorstehenden Ansprüche, **dadurch gekennzeichnet, dass** weitere Peelingpartikel, enthaltend wachsartige Substanzen enthalten sind.

5. Zubereitung nach wenigstens einem der vorstehenden Ansprüche, **dadurch gekennzeichnet, dass** die wachsartigen Substanzen ausgewählt werden aus der Gruppe Carnaubawachs, Candellila Wachs, Sonnenblumenwachs, gehärtetes Rizinusöl, Reiswachs, gehärtetes Sonnenblumenöl, gehärtetes Sojaöl, Hartparaffin, mikrokristallines Wachs, synthetisches Wachs, gehärtetes Jojobawachs und Stearyl Stearat.

6. Zubereitung nach Anspruch 4, **dadurch gekennzeichnet, dass** das Gewichtsverhältnis der Peelingpartikel bestehend aus mindestens einer im Wesentlichen natürlichen oder naturbasierten Substanz, insbesondere aus mindestens einer Substanz ausgewählt aus der Gruppe, Hydrated Silica, Polymilchsäure und mikrokristalliner Cellulose zu Peelingpartikeln bestehend aus mindestens einer wachsartige Substanz 1:10 bis 10:1, bevorzugt 3:2 bis 2:3 beträgt.

7. Zubereitung nach wenigstens einem der vorstehenden Ansprüche, **dadurch gekennzeichnet, dass** die Tenside ausgewählt werden aus der Gruppe der anionischen, amphoteren und nichtionischen Tenside.

8. Zubereitung nach wenigstens einem der vorstehenden Ansprüche, **dadurch gekennzeichnet, dass** die anionischen Tenside Alkali- und/oder Ammoniumsalze der Alkylsulfate und/oder Alkylethersulfate sind, wobei die Alkylsulfate und/oder Alkylethersulfate Alkylreste mit einer Kettenlänge von 8 bis 16 Kohlenstoffatomen, insbesondere Alkylreste mit einer Kettenlänge von 10 bis 14 Kohlenstoffatomen und ganz insbesondere Alkylreste mit einer Kettenlänge von 11 bis 13 Kohlenstoffatomen, aufweisen.

9. Zubereitung nach wenigstens einem der vorstehenden Ansprüche, **dadurch gekennzeichnet, dass** die amphoteren Tenside Alkyl- und/oder Alkylamidopropylbetaine sind, wobei die Alkyl- und/oder Alkylamidopropylbetaine Alkylreste mit einer Kettenlänge von 8 bis 16 Kohlenstoffatomen, insbesondere Alkylreste mit einer Kettenlänge von 10 bis 14 Kohlenstoffatomen und ganz insbesondere Alkylreste mit einer Kettenlänge von 11 bis 13 Kohlenstoffatomen, aufweisen.

10. Zubereitung nach wenigstens einem der vorstehenden Ansprüche, **dadurch gekennzeichnet, dass** die Alkyl- und/oder Alkylamidopropylbetaine Alkylgruppen aufweisen, die aus der Aufarbeitung von Kokosöl zu Tensiden stammen.

11. Zubereitung nach Anspruch 10, **dadurch gekennzeichnet, dass** die Alkyl- und/oder Alkylamidopropylbetaine Cocco-Betaine und/oder Cocoamidopropylbetaine sind.

12. Zubereitung nach wenigstens einem der vorstehenden Ansprüche, **dadurch gekennzeichnet, dass** die nichtionischen Tenside ethoxilierte Glycerinester sind.

13. Zubereitung nach wenigstens einem der vorstehenden Ansprüche, **dadurch gekennzeichnet, dass** die ethoxilierten Glycerinester Alkylreste mit einer Kettenlänge von 8 bis 16 Kohlenstoffatomen, insbesondere 10 bis 14 Kohlenstoffatomen, ganz insbesondere 11 bis 13 Kohlenstoffatomen aufweisen.

14. Zubereitung nach wenigstens einem der vorstehenden Ansprüche, **dadurch gekennzeichnet, dass** die ethoxilierten Glycerinester PEG-7 Glyceryl Cocoate sind.

15. Zubereitung nach wenigstens einem der vorstehenden Ansprüche, **dadurch gekennzeichnet, dass** die Zubereitungen frei sind von halogenorganischen Konservierungsmitteln.

16. Zubereitung nach wenigstens einem der vorstehenden Ansprüche, **dadurch gekennzeichnet, dass** die Zubereitungen frei sind von Paraben-haltigen Konservierungsmitteln aufweisend eine Alkylseitenkette von mehr als 3 Kohlenstoffatomen.

17. Zubereitung nach wenigstens einem der vorstehenden Ansprüche, **dadurch gekennzeichnet, dass** die Zubereitungen frei sind von Paraben-haltigen Konservierungsmitteln ausgewählt aus der Gruppe Methyl-, Ethyl-, Propyl-, Isopropyl- Isobutyl-, Pentyl- und Phenylparaben.

## Claims

1. Skin cleansing preparations comprising surfactants and peeling particles, wherein the peeling particles consist of at least one substance selected from the group comprising hydrated silica, polylactic acid and microcrystalline cellulose,
wherein the preparations are free from formaldehyde releasing preservatives, 2-bromo-2-nitropropane-1,3-diol and butylparaben,
wherein benzoic acid and/or a salt of benzoic acid are present as preservative and
wherein the content of peeling particles in the cleansing preparations is 0.075% to 7% by weight, based on the total weight of the preparation.

2. Cleansing preparations according to Claim 1, **characterized in that** the peeling particles in each case consist of an essentially natural or nature-based substance, in particular in each case only hydrated silica or polylactic acid or microcrystalline cellulose.

3. Cleansing preparations according to Claim 1, **characterized in that** the preparations comprise a mixture of peeling particles, wherein the peeling particles consist of essentially natural or nature-based substances, particularly of substances selected from the group comprising hydrated silica, polylactic acid and microcrystalline cellulose.

4. Preparation according to at least one of the preceding claims, **characterized in that** further peeling particles comprising waxy substances are present.

5. Preparation according to at least one of the preceding claims, **characterized in that** the waxy substances are selected from the group comprising carnauba wax, candelilla wax, sunflower wax, hardened castor oil, rice wax, hardened sunflower oil, hardened soya oil, hard paraffin, microcrystalline wax, synthetic wax, hardened jojoba wax and stearyl stearate.

6. Preparation according to Claim 4, **characterized in that** the ratio by weight of the peeling particles consisting of at least one essentially natural or nature-based substance, particularly of at least one substance selected from the group comprising hydrated silica, polylactic acid and microcrystalline cellulose, to peeling particles consisting of at least one waxy substance is from 1:10 to 10:1, preferably from 3:2 to 2:3.

7. Preparation according to at least one of the preceding claims, **characterized in that** the surfactants are selected from the group of anionic, amphoteric and non-ionic surfactants.

8. Preparation according to at least one of the preceding claims, **characterized in that** the anionic surfactants are alkali metal and/or ammonium salts of alkyl sulfates and/or alkyl ether sulfates, wherein the alkyl sulfates and/or alkyl ether sulfates comprise alkyl radicals having a chain length of 8 to 16 carbon atoms, particularly alkyl radicals having a chain length of 10 to 14 carbon atoms and more particularly alkyl radicals having a chain length of 11 to 13 carbon atoms.

9. Preparation according to at least one of the preceding claims, **characterized in that** the amphoteric surfactants are alkyl- and/or alkyl amidopropyl betaines, wherein the alkyl- and/or alkyl amidopropyl betaines comprise alkyl radicals having a chain length of 8 to 16 carbon atoms, particularly alkyl radicals having a chain length of 10 to 14 carbon atoms and more particularly alkyl radicals having a chain length of 11 to 13 carbon atoms.

10. Preparation according to at least one of the preceding claims, **characterized in that** the alkyl- and/or alkyl amidopropyl betaines comprise alkyl groups originating from the processing of coconut oil to surfactants.

11. Preparation according to Claim 10, **characterized in that** the alkyl- and/or alkyl amidopropyl betaines are coco betaine and/or cocamidopropyl betaine.

12. Preparation according to at least one of the preceding claims, **characterized in that** the non-ionic surfactants are ethoxylated glycerin esters.

13. Preparation according to at least one of the preceding claims, **characterized in that** the ethoxylated glycerin esters comprise alkyl radicals having a chain length of 8 to 16 carbon atoms, particularly 10 to 14 carbon atoms, more particularly 11 to 13 carbon atoms.

14. Preparation according to at least one of the preceding claims, **characterized in that** the ethoxylated glycerin esters are PEG-7 glyceryl cocoate.

15. Preparation according to at least one of the preceding claims, **characterized in that** the preparations are free from halo-organic preservatives.

16. Preparation according to at least one of the preceding claims, **characterized in that** the preparations are free from paraben-containing preservatives comprising an alkyl side chain of more than 3 carbon atoms.

17. Preparation according to at least one of the preceding claims, **characterized in that** the preparations are free from paraben-containing preservatives selected from the group comprising methyl-, ethyl-, propyl-, isopropyl-, isobutyl-, pentyl- and phenylparaben.

## Revendications

1. Préparations nettoyantes de la peau contenant des tensioactifs et des particules d'exfoliation, les particules d'exfoliation étant constituées par au moins une substance choisie dans le groupe formé par la silice hydratée, le poly(acide lactique) et la cellulose microcristalline, les préparations étant exemptes de conservateurs dissociant du formaldéhyde, de 2-bromo-2-nitropropane-1,3-diol et de butylparabène, les préparations contenant de l'acide benzoïque et/ou un sel de l'acide benzoïque en tant que conservateur et la teneur en particules d'exfoliation dans les préparations nettoyantes étant de 0,075 à 7% en poids, par rapport au poids total de la préparation.

2. Préparations nettoyantes selon la revendication 1, **caractérisées en ce que** les particules d'exfoliation ne sont à chaque fois constituées que par une seule substance sensiblement naturelle ou à base naturelle, en particulier à chaque fois uniquement par de la silice hydratée ou du poly(acide lactique) ou de la cellulose microcristalline.

3. Préparations nettoyantes selon la revendication 1, **caractérisées en ce que** les préparations contiennent un mélange de particules d'exfoliation, les particules d'exfoliation étant constituées par des substances sensiblement naturelles ou à base naturelle, en particulier par des substances choisies dans le groupe formé par la silice hydratée, le poly(acide lactique) et la cellulose microcristalline.

4. Préparation selon au moins l'une quelconque des revendications précédentes, **caractérisée en ce que** d'autres particules d'exfoliation contenant des substances de type cire sont contenues.

5. Préparation selon au moins l'une quelconque des revendications précédentes, **caractérisée en ce que** les substances de type cire sont choisies dans le groupe formé par la cire de carnauba, la cire de candelilla, la cire de tournesol, l'huile de ricin hydrogénée, la cire de riz, l'huile de tournesol hydrogénée, l'huile de soja hydrogénée, la paraffine dure, la cire microcristalline, la cire synthétique, la cire de jojoba hydrogénée et le stéarate de stéaryle

6. Préparation selon la revendication 4, **caractérisée en ce que** le rapport pondéral des particules d'exfoliation, constituées par au moins une substance sensiblement naturelle ou à base naturelle, en particulier par au moins une substance choisie dans le groupe formé par la silice hydratée, le poly(acide lactique) et la cellulose microcristalline, aux particules d'exfoliation constituées par au moins une substance de type cire est de 1:10 à 10:1, de préférence de 3:2 à 2:3.

7. Préparation selon au moins l'une quelconque des revendications précédentes, **caractérisée en ce que** les tensioactifs sont choisis dans le groupe des tensioactifs anioniques, amphotères et non ioniques.

8. Préparation selon au moins l'une quelconque des revendications précédentes, **caractérisée en ce que** les tensioactifs anioniques sont des sels de métal alcalin et/ou d'ammonium d'alkylsulfates et/ou d'alkyléthersulfates, les alkylsulfates et/ou les alkyléthersulfates présentant des radicaux alkyle d'une longueur de chaîne de 8 à 16 atomes de carbone, en particulier des radicaux alkyle d'une longueur de chaîne de 10 à 14 atomes de carbone et tout particulièrement des radicaux alkyle d'une longueur de chaîne de 11 à 13 atomes de carbone.

9. Préparation selon au moins l'une quelconque des revendications précédentes, **caractérisée en ce que** les tensioactifs amphotères sont des alkylbétaïnes et/ou des alkylamidopropylbétaïnes, les alkylbétaïnes et/ou les alkylamidopropylbétaïnes présentant des radicaux alkyle d'une longueur de chaîne de 8 à 16 atomes de carbone, en particulier des radicaux alkyle d'une longueur de chaîne de 10 à 14 atomes de carbone et tout particulièrement des radicaux alkyle d'une longueur de chaîne de 11 à 13 atomes de carbone.

10. Préparation selon au moins l'une quelconque des revendications précédentes, **caractérisée en ce que** les alkylbétaïnes et/ou les alkylamidopropylbétaïnes présentent des groupes alkyle qui proviennent du traitement d'huile de coco en tensioactifs.

11. Préparation selon la revendication 10, **caractérisée en ce que** les alkylbétaïnes et/ou les alkylamidopropylbétaïnes sont des bétaïnes de coco et/ou des cocoamidopropylbétaïnes.

12. Préparation selon au moins l'une quelconque des revendications précédentes, **caractérisée en ce que** les tensioactifs non ioniques sont des esters de glycérol éthoxylés.

13. Préparation selon au moins l'une quelconque des revendications précédentes, **caractérisée en ce que** les esters de glycérol éthoxylés présentent des radicaux alkyle d'une longueur de chaîne de 8 à 16 atomes de carbone, en particulier de 10 à 14 atomes de carbone et tout particulièrement de 11 à 13 atomes de carbone.

14. Préparation selon au moins l'une quelconque des revendications précédentes, **caractérisée en ce que** les esters de glycérol éthoxylés sont des cocoates de PEG-7-glycéryle.

15. Préparation selon au moins l'une quelconque des revendications précédentes, **caractérisée en ce que** les préparations sont exemptes de conservateurs halogéno-organiques.

16. Préparation selon au moins l'une quelconque des revendications précédentes, **caractérisée en ce que** les préparations sont exemptes de conservateurs contenant des parabènes présentant une chaîne alkyle latérale de plus de 3 atomes de carbone.

17. Préparation selon au moins l'une quelconque des revendications précédentes, **caractérisée en ce que** les préparations sont exemptes de conservateurs contenant des parabènes choisis dans le groupe comprenant le méthylparabène, l'éthylparabène, le propylparabène, l'isopropylparabène, l'isobutylparabène, le pentylparabène et le phénylparabène.
